(19) Europäisches Patentamt / European Patent Office / Office européen des brevets

(11) **EP 1 834 582 A1**

(12) **EUROPEAN PATENT APPLICATION**

(43) Date of publication:
**19.09.2007 Bulletin 2007/38**

(51) Int Cl.:
**A61B 5/15** (2006.01)

(21) Application number: **07104285.7**

(22) Date of filing: **16.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **16.03.2006 JP 2006072986**

(71) Applicant: **Fujifilm Corporation**
**Minato-ku**
**Tokyo (JP)**

(72) Inventors:
• **Tashiro, Tomoko**
**c/o Fujifilm Corporation**
**Asaka-shi, Saitama (JP)**
• **Sakaino, Yoshiki**
**c/o Fujifilm Corporation**
**Asaka-shi, Saitama (JP)**
• **Abe, Yoshihiko**
**c/o Fujifilm Corporation**
**Asaka-shi, Saitama (JP)**

(74) Representative: **Banzer, Hans-Jörg**
**Kraus & Weisert**
**Patent- und Rechtsanwälte**
**Thomas-Wimmer-Ring 15**
**80539 München (DE)**

(54) **Blood collecting needle, syringe needle, winged needle, test kit and blood collecting kit**

(57)    A blood collecting needle, which comprises: a needle tube having a human-body puncturing needle tube and a stopper-body puncturing needle tube (3), wherein the needle tube has an outer diameter having substantially the same dimension from a tip (5) of the human-body puncturing needle tube (3) to a tip (6) of the stopper-body puncturing needle tube (3), the needle tube has an inner diameter having substantially the same dimension from the tip (5) of the human-body puncturing needle tube (1) to the tip (6) of the stopper-body puncturing needle tube (3), and the blood collecting needle satisfies equation (1):

$0.35 \leq D_1 < 0.70$ (equation 1)

wherein $D_1$ represents the outer diameter (mm) of the needle tube.

## FIG. 1

**Description**

**Background of the Invention**

1. Field of the Invention

The present invention relates to a blood collecting needle, a syringe needle, a winged needle, a test kit and a blood collecting kit having the same.

2. Description of the Related Art

A method of diagnosing diseases of humans with blood, urine, etc. being used as analytes has been hitherto performed for a long time as a method that can simply diagnose human bodies without damaging the human bodies.

Particularly with respect to blood, diagnosis for many test items can be performed, and it is general in health examination, diagnosis of diseases, etc. to collect blood and analyze the components of the blood.

In the blood test, when blood is collected from a so-called examinee such as a patient, a health examination target or the like, blood is normally collected according to a vacuum blood collecting method or a syringe blood colleting method when. The vacuum blood collecting method for sucking blood by using the negative pressure of a vacuum blood colleting tube has been broadly used because the time required for blood collection is short and also hemolysis hardly occurs. In this case, it is general to collect blood by using a blood collecting needle. Only 21G (gauge) (0.81mm in outer diameter, 0.49mm to 0.61mm in inner diameter) or 22G (gauge) (0.70mm in outer diameter, 0. 39mm to 0. 52mm in inner diameter) which is defined in ISO9626: 1991 or the like is placed on the market as an ordinary blood collecting needle for vacuum collection, and it is normal that pain occurs when the needle stings into vein. Furthermore, the syringe blood collecting method in which a blood collector collects blood by pulling an inner cylinder has a disadvantage that blood collection requires much time. However, this method can collect blood in accordance with a required amount of blood or the condition of a blood collection target and thus it is used for babies, elderly people, etc. from who a large amount of blood cannot be collected. The blood collecting needle of the present invention is used for the vacuum blood collection, and a syringe needle and a winged needle can be used in both the vacuum blood collection and the syringe blood collection.

As means of solving the problem at the puncturing time, JP-2004-41391 discloses a technique of reducing pain at the puncturing time by using a needle having a narrow tip and a thick base.

Furthermore, as an intravascular detaining needle for injecting drug solution, JP-A-2-65870 discloses a needle having a side hole to secure a flow amount while using a small-diameter needle having a straight shape.

Furthermore, JP-T-2001-527435 discloses a needle for injecting medical substances which is characterized in that the inner diameter of the tube thereof is narrower than the size defined in ISO9626:1991.

**Summary of the Invention**

In the technique of the JP-2004-41391, the pain at the puncturing time is reduced, and it seems to be expected that the flow amount of blood at the blood collection time can be secured by applying this technique to blood collection. However, this technique adopts a structure that the needle is narrowed down, so that there is a problem that the processing cost of a hollow tube as a needle material increases and thus it is difficult to supply cheap needles.

When the technique of the JP-A-2-65870 is used, the liquid resistance in the tube is defined by the inner diameter of the tube and thus a large effect is hardly achieved in securement of the flow rate. Furthermore, the needle is produced by providing a step of forming a side hole in the needle, and thus it is difficult to supply cheap needles.

The size of the puncturing needle disclosed in the JP-T-2001-527435 is set to be less than 9mm in length, less than 0. 320mm in outer diameter and larger than 0 . 165mm in inner diameter, or to be less than 9mm in length, less than 0.298mm in outer diameter and larger than 0.133mm in inner diameter. However, this technique aims at administration of insulin, and there is no description on the blood collection and the blood collecting needle.

An object of the present invention is to provide a blood collecting needle that can reduce pain at the blood collecting time as compared with a normal blood collecting needle. Furthermore, an object of the present invention is to provide a method of efficiently designing a blood collecting needle, a syringe needle and a winged needle with which pain is suppressed and the flow rate of blood is high.

The above obj ects of the present invention can be attained by the following construction.

(1) A blood collecting needle, which comprises:

a needle tube having a human-body puncturing needle tube and a stopper-body puncturing needle tube,

wherein the needle tube has an outer diameter having substantially the same dimension from a tip of the human-body puncturing needle tube to a tip of the stopper-body puncturing needle tube,
the needle tube has an inner diameter having substantially the same dimension from the tip of the human-body puncturing needle tube to the tip of the stopper-body puncturing needle tube, and
the blood collecting needle satisfies equation (1):

$$0.35 \leq D_1 < 0.70 \qquad \text{(equation 1)}$$

wherein $D_1$ represents the outer diameter (mm) of the needle tube.
(2) The blood collecting needle as described in (1) above, which satisfies equation (2):

$$0.9 \times D_1 - 0.1 \leq D_2 \leq 0.9 \times D_1 - 0.04 \qquad \text{(equation 2)}$$

wherein $D_1$ represents the outer diameter (mm) of the needle tube; and
$D_2$ represents the inner diameter (mm) of the needle tube.
(3) A syringe needle, which comprises:

a needle tube having a tip and a base,

wherein the needle tube has an outer diameter having substantially the same dimension from the tip to the base of the needle tube,
the needle tube has an inner diameter having substantially the same dimension from the tip to the base of the needle tube, and
the syringe needle satisfies equation (1) and equation (2):

$$0.35 \leq D_1 < 0.70 \qquad \text{(equation 1)}$$

$$0.9 \times D_1 - 0.1 \leq D_2 \leq 0.9 \times D_1 - 0.04 \qquad \text{(equation 2)}$$

wherein $D_1$ represents the outer diameter (mm) of the needle tube; and
$D_2$ represents the inner diameter (mm) of the needle tube.
(4) A winged needle, which comprises:

a needle tube having a tip and a base,

wherein the needle tube has an outer diameter having substantially the same dimension from the tip to the base of the needle tube,
the needle tube has an inner diameter having substantially the same dimension from the tip to the base of the needle tube, and
the winged needle satisfies equation (1) and equation (2) :

$$0.35 \leq D_1 < 0.70 \qquad \text{(equation 1)}$$

$$0.9 \times D_1 - 0.1 \leq D_2 \leq 0.9 \times D_1 - 0.04 \qquad \text{(equation 2)}$$

wherein $D_1$ represents the outer diameter (mm) of the needle tube; and

$D_2$ represents the inner diameter (mm) of the needle tube.

(5) The blood collecting needle, the syringe needle or the winged needle as described in any of (1) to (4) above, which further comprises:

a needle base,

wherein the needle base has a mark indicating an orientation of a blade.

(6) The blood collecting needle, the syringe needle or the winged needle as described in any of (1) to (5) above, which satisfies equation (3):

$$0.9 \times D_1 - 0.09 \leq D_2 \leq 0.9 \times D_1 - 0.05 \quad (\text{equation } 3)$$

wherein $D_1$ represents the outer diameter (mm) of the needle tube; and

$D_2$ represents the inner diameter (mm) of the needle tube.

(7) A test kit, which comprises:

the blood collecting needle, the syringe needle or the winged needle as described in any of (1) to (6) above; and a test chip.

(8) A blood collecting kit, which comprises:

the blood collecting needle, the syringe needle or the winged needle as described in any of (1) to (6) above; and a vacuum blood collecting tube.

(9) The blood collecting kit as described in (8) above,

wherein the vacuum blood collecting tube has a content amount of 2.5mL or less.

**Brief Description of the Drawings**

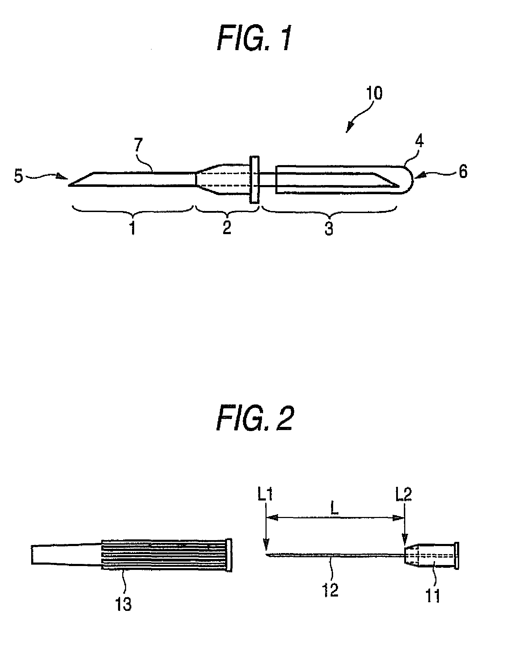

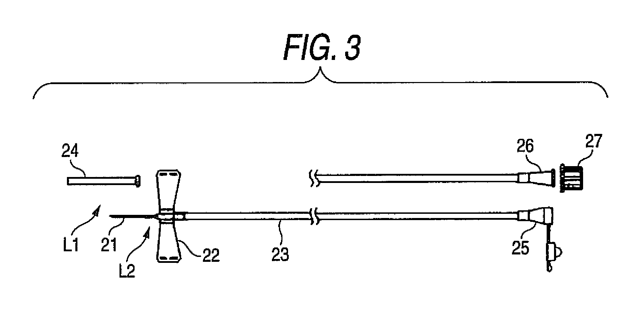

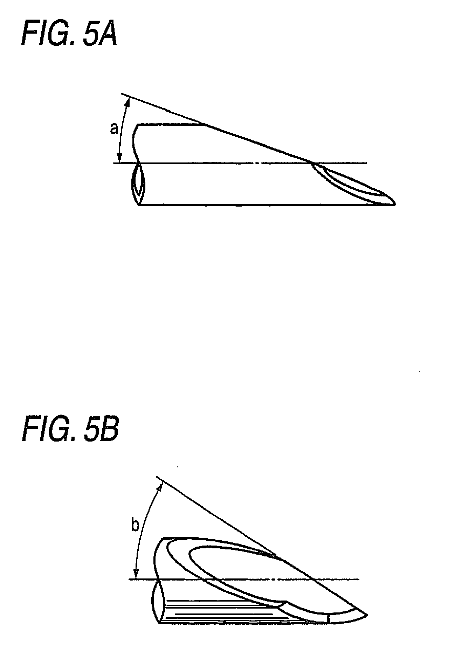

Fig. 1 is a diagram showing an exemplary embodiment of a blood collecting needle of the present invention;
Fig. 2 is a diagram showing an exemplary embodiment of a syringe needle according to the present invention;
Fig. 3 is a diagram showing an exemplary embodiment of a winged needle according to the present invention;
Fig. 4 is a diagram showing the dimension of the needle of the present invention; and
Fig. 5A and 5B are diagrams showing an embodiment of a cut shape of the needle point of the needle of the present invention,

wherein in Fig. 1, 1 denotes human -body puncturing needle tube; 2 denotes needle base (hub); 3 denotes stopper-body puncturing needle tube; 4 denotes blood stanching cover; 5 denotes tip of human-body puncturing side; 6 denotes tip of stopper-body puncturing side; and 7 denotes needle tube (cannula) ; in Fig. 2, 11 denotes needle base (hub); 12 denotes needle tube (cannula); 13 denotes protection cap; L1 denotes tip of needle tube; and L2 denotes base of needle tube; in Fig. 3, 21 denotes needle tube (cannula); 22 denotes needle base (hub); 23 denotes conduit tube; 24 denotes protection cap; 25 denotes female engaging portion (with cap) ; 26 denotes female engaging portion; 27 denotes cap; L1 denotes tip of needle tube; and L2 denotes base of needle tube; and in Fig. 5, a denotes first grinding angle; and b denotes second grinding angle.

**Detailed Description of the Invention**

The present invention will be further described in detail hereunder.

<Use obj ect of blood collecting needle, syringe needle, winged needle>

A blood collecting needle, a syringe needle and a winged needle of the present invention are properly used when blood of a human being and other animals is collected. The collected blood is normally used for blood test.

<Blood Collection Method>

The blood collection is carried out by two kinds of methods of normal syringe blood collection and vacuum blood collection. The syringe blood collection is based on a method of fitting a needle to a syringe and collecting blood, and it is frequently used to collect a small amount of blood of animals, babies, etc. This method has a problem that the blood collecting time is long and hemolysis easily occurs. On the other hand, the vacuum blood collection is based on a method of fitting a blood collecting needle to a vacuum blood collecting holder and collecting blood by negative pressure of a blood collecting tube. The blood collecting needle of the present invention is a blood collecting needle used for the latter vacuum blood collecting method. The syringe needle and the winged needle of the present invention can be used in both the syringe blood collection and the vacuum blood collection.

<Classification of Puncturing Needle>

There are a hollow needle and a non-hollow needle as a needle for collecting blood from an arm, an elbow, a fingertip or the like to collect blood and analyze components in the blood.
For example, the hollow puncturing needle is generally punctured into vein to collect blood in vein and normally used to analyze components in the blood in a medical institution or for health examination. For example, it is used to analyze components for diagnosing glucosuria such as blood sugar level, hemoglobinAlc (HbAlc), etc., components for diagnosing the kidney function such as glutamate pyruvate transaminase (GPT), glutamate oxaloacetate transaminase (GOT), etc., and components for diagnosing the renal function such as creatinine (CRE), urea nitrogen (BUN), etc.
On the other hand, the non-hollow puncturing needle called as lancet is generally used for stinging a fingertip by an examinee himself/herself to collect his/her blood.
The present invention relates to a blood collecting needle, a syringe needle and a winged needle whose insides are hollow. The first mode of the present invention relates to the blood collecting needle, the second mode relates to the syringe needle, and the third mode relates to the winged needle.

<Blood Collecting Needle>

First, the blood collecting needle corresponding to the first mode of the present invention will be described. Fig. 1 shows a general structure of the blood collecting needle 10. It comprises a needle tube (cannula) 7 having blade edges at both the ends penetrating in the forward and backward direction, and a needle base (hub). In Fig. 1, 1 represents a human-body puncturing needle tube, 2 represents a needle base (hub), and 3 represents a stopper-body puncturing needle tube. For continuous blood collection, the needle is equipped with a blood stanching rubber cover (rubber sleeve) 4 fitted to the rear portion of the stopper-body puncturing side. Normally, the blood collecting needle is generally placed on the market under the state that it is covered by a protection cap (protector) for protecting edges. After the blood collecting needle is mounted in a holder for vacuum blood collection so that the recess side of the holder faces the stopper-body puncturing needle tube side, the human-body puncturing needle tube is punctured into vein, and further a vacuum blood collecting tube is inserted into the holder, whereby the stopper-body puncturing needle tube is punctured into the stopper body of the vacuum blood collecting tube and blood collection is started. Normally, the blood collecting needle is manufactured according to a method in which both the ends of one needle tube are polished to create edges, a needle base is jointed to the center portion of the needle tube and then adhesively attached to the needle tube, and then the joint result is covered by a blood stanching cover. The tip of the needle tube at the human-body puncturing side is a side represented by 5 in Fig. 1, and the tip of the stopper-body puncturing needle is a side represented by 6 in Fig. 1. General styles are defined in JIST-3220.

<Syringe Needle>

Next, the syringe needle corresponding to the second mode of the present invention will be described. The syringe needle is used for all fields such as hypodermic injection, intramuscular injection, etc. as well as blood collection, and it is general to select the girth of the syringe needle in accordance with a used application. As shown in Fig. 2, the syringe needle comprises a needle base (hub) 11 and a needle tube (cannula) 12. Normally, it is placed on the market while covered by a protection cap (protector) 13 for protecting edges. A method of joining the needle to a syringe for blood collection and a method of performing vacuum blood collection by using a lure adaptor are known. General styles are defined in JIS T-3209.

<Winged needle>

The winged needle corresponding to the third mode of the present invention will be described. The winged needle is a vein needle having a conduit tube which is normally used to perform blood transfusion within several hours. It has a winged needle base for fixing the vein needle to the skin. It may be used merely for blood collection. For example, when there is a high risk that a blood collection target moves at the blood collection time like an animal, a baby or the like, the risk that the needle drops out or is misaligned is reduced by using the winged needle, and thus this needle is suitable. When many blood collecting tubes are used in the vacuum blood collection, replacement of the blood collecting tube can be easily performed by using the winged needle.

As shown in Fig. 3, the winged needle comprises a needle base (hub) 22 having fixing wings, a needle tube (cannula) 21, and a conduit tube 23. A side of the needle to be fitted to a syringe or vacuum blood collecting holder is provided with a female engaging portion 25 or 26 which is matched with the taper of the syringe, and there is a cap integral type 25 or a cap separately-mounted type 26. In the case of 26, the needle is placed on the market while a cap 27 is put on the needle. Furthermore, normally, the needle is placed on the market while covered by a protection cap (protector) 24 for protecting edges. When the needle is put on the vacuum blood collecting holder, it is used after a lure adaptor is fitted. There is sold such a type that the lure adaptor is integrated with the needle in advance, and thus it is usable directly for vacuum blood collection. The winged needle of the present invention may be designed in any shape. It is preferable that the needle is integrated with the lure adaptor so that it is easily usable. General styles are defined in JIS T-3222.

Normally, only blood collecting needles from 20G to 22G are placed on the market. Even when blood collection is performed by a syringe needle or a winged needle, 21G (0.80mm in outer diameter) or 22G (0.70mm in outer diameter) is generally used.

The blood collecting needle of the present invention is designed to have substantially the same dimension in outer diameter ($D_1$) from the tip of the human-body puncturing needle tube to the tip of the stopper-body puncturing needle tube, and also have substantially the same dimension in inner diameter ($D_2$) from the tip of the human-body puncturing needle tube to the tip of the stopper-body puncturing needle tube. In Fig. 1, 5 represents the tip of the human-body puncturing needle tube, and 6 represents the tip of the stopper-body puncturing needle tube.

Furthermore, the syringe needle or the winged needle of the present invention is designed to have substantially the same dimension in outer diameter ($D_1$) from the tip to the base and also have substantially the same dimension in inner diameter ($D_2$ from the tip to the base. The tip of the needle tube is defined by L1 in Figs. 2 and 3, and the base of the needle tube is defined by L2 in Figs. 2 and 3.

Furthermore, "substantially the same dimension" means a dimension achieved when no intentional dimensional change such as tapering or the like is made and the manufacturing is carried out with an intention of creating the same dimension. In the needle of the present invention, it corresponds to the dimension of each of the outer diameter and the inner diameter which is set to the range of ±0.1mm, more preferably ±0.07mm from the tip to the base except for the tip portion of the needle cut out for puncturing.

By setting substantially the same dimension, the processing cost can he suppressed, and the manufacturing can be performed at low price.

The dimension of the blood collecting needle, the syringe needle or the winged needle defined in this specification indicates the dimension of the needle tube (cannula) portion of the needle.

The outer diameter ($D_1$) of the needle tube of the present invention satisfies the equation 1. $0.35 \leq D_1 < 0.70$ (equation 1). That is, the outer diameter is not less than 0.35mm, and smaller than 0.70mm. If the outer diameter is not less than 0.70mm, the pain at the blood collecting time gets heavy. The outer diameter is preferable smaller than 0.70mm, more preferably equal to 0.65mm or less, and most preferable equal to 0. 60mm or less. If the outer diameter is less than 0. 35mm, the physical strength of the needle is weak, and it obstructs blood collection. The outer diameter is preferably equal to 0.35mm or more, and more preferably equal to 0.40mm or more.

When the inner diameter ($D_2$) of the needle is increased, the blood collecting time is shortened, and the pain of the blood collecting target can be reduced. However, if the inner diameter is excessively increased, the radial thickness of the needle is thinner, so that the physical strength of the needle is weakened and thus it is difficult to carry out blood collection. If the outer diameter of the needle is increased, the minimum radial thickness required to keep the strength is increased, and if the outer diameter of the needle is narrowed, the radial thickness required to keep the strength is reduced. That is, the optimum radial thickness corresponding to the outer diameter exists, and the inner diameter must be designed in conformity wit the optimum radial thickness. As a result of detailed considerations, when the outer diameter is determined, a method (calculation equation) for calculating the optimum inner diameter in consideration with the blood collecting time and the strength of the needle has been found. By carrying out the designing using this equation, a conventional cumbersome work of making a prototype and checking the physical strength and the blood collecting time thereof can be omitted, and the efficient design can be performed.

That is, with respect to the blood collecting needle of the present invention, it is preferable that the outer diameter and the inner diameter are designed so as to satisfy $0.9 \times D_1 - 0.1 \leq D_2 \leq 0.9 \times D_1 - 0.04$ (equation 2). If the inner diameter $D_2$ is smaller than $0.9 \times D_1 - 0.1$, the blood collecting speed is late. Furthermore, if the inner diameter $D_2$ is larger than $0.9 \times D_1 - 0.04$, the strength of the needle is insufficient. It is preferable that $0.9 \times D_1 - 0.1 \leq D_2 \leq 0.9 \times D_1$

- 0.04, and it is more preferable that $0.9 \times D_1 - 0.09 \leq D_2 \leq 0.9 \times D_1 - 0.05$ (equation 3). In the equation, the numerical value (0.9) corresponding to the gradient is multiplied to the outer diameter so that the outer diameter is corrected to be slightly small. Furthermore, the radial thickness of the needle is subtracted from the reduced outer diameter by the portion (0.1, 0.04) corresponding to the Y intercept.

The syringe needle and the winged needle of the present invention surely satisfies $0.9 \times D_1 - 0.1 \leq D_2 \leq 0.9 \times D_1 - 0.04$ (equation 2). The preferable range is the same as the blood collecting needle described above.

The length of the human-body puncturing needle tube of the blood collecting needle of the present invention preferably ranges from 5mm to 40mm, more preferably from 7mm to 35mm, and most preferably from 9mm to 30mm. The length of the human-body puncturing needle tube means the length of the human-body puncturing needle tube except for the portion hidden by the needle base.

The length of the stopper-body puncturing needle tube of the blood collecting needle of the present invention is required to be so sufficient that it penetrates through the stopper-body of the vacuum blood collecting tube, and thus it is better that the needle tube is as long as possible. However, it is better that the length of the needle tube is as small as possible to prevent the tip portion of the stopper-body puncturing needle tube from coming into contact with blood stocked in the blood collecting tube. The length of the stopper-body puncturing needle tube preferably ranges from 10mm to 25mm, more preferably from 12mm to 22mm, and most preferably from 14mm to 20mm. The length of the stopper-body puncturing needle tube means the length of the stopper-body puncturing needle tube except for the portion hidden by the needle base.

The overall length of the blood collection needle of the present invention corresponds to the sum of the length of the human-body puncturing needle tube, the length of the stopper-body puncturing needle tube and the length of the needle base. It preferably ranges from 20mm to 70mm, and more preferably ranges from 30mm to 60mm.

The length of the needle tube of the syringe needle or winged needle of the present invention preferably ranges from 5mm to 40mm, more preferably from 7mm to 35mm, and most preferably from 9mm to 30mm. The length of the needle tube is the length of the needle tube except for the portion hidden by the needle base, and it indicates the length from L1 to L2.

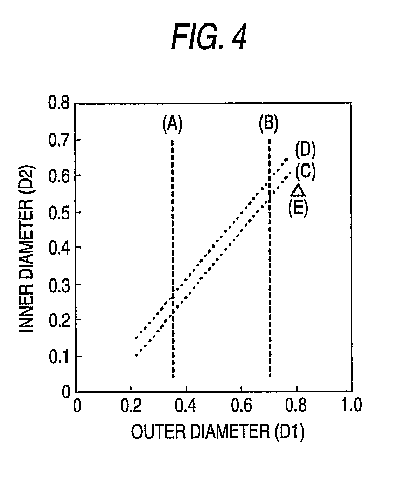

Fig. 4 shows the dimensions shown in the equation 1 and the equation 2. (A) shows the lower limit of the equation 1, (B) shows the upper limit of the equation 1, (C) shows the lower limit of the equation 2, and (D) shows the upper limit of the equation 2, and (E) shows an example of the dimension of 21G generally used for blood collection. The blood collecting needle of the present invention has a dimension in the range from (A) to (B), preferably enters the range surrounded by (A), (B), (C) and (D). The syringe needle or the winged needle of the present invention enters the range surrounded by (A), (B), (C), (D).

The needle of the present invention has an outer diameter smaller than the needle of 21G which is normally used for blood collection, and thus the orientation of the edge of the needle may be hard to see. Therefore, it is preferable to provide the needle base with a mark indicating the orientation of the blade. Any mark such as Δ O ●, etc. may be provided as the mark. The mark may be provided by any method such as print, seal attachment, recessing/projecting, or the like.

(Cut of Needle)

Furthermore, any one of three-sides cutting or one-side cutting, linear cutting or curved-line cutting may be used as the cutting shape of the needlepoint for puncturing. The cutting angle of the needlepoint in the case of the one-side cutting is preferably in the range from 8 degrees to 30 degrees in the angle θ of the blade edge defined in Japanese Industrial Standards T3101-1979. The cutting angle in the case of the three-side cutting is preferably set so that the first grinding angle a is in range from 7 degrees to 20 degrees and the second grinding angle b is in the range from 10 degrees to 30 degrees. Figs. 5A and 5B show a preferable three-side cut needlepoint. The first grinding angle a is shown in Fig. 5A, and the second grinding angle b is shown in Fig. 5B (see ISO7864).

(Material of Needle)

The material of the needle of the present invention is not limited to a special material, and any material such as metal such as stainless steel, nickel free stainless or the like of SUS304 or the like, resin such as polycarbonate, acrylate polymer or the like, inorganicmaterial such as quartz glass, boron silicate glass or the like may be used as insofar as a hollow needle can be formed of the material concerned. Among these materials, metal such as stainless steel or the like is preferably used because it is easily to keep the dynamical strength under the above outer diameter. Furthermore, the outer surface or inner surface of the needle may be subjected to a treatment such as coating or the like by using a material different from the material of the needle. Particularly, another material (silicon oil or the like) may be coated to reduce the puncturing resistance and facilitate puncturing.

Furthermore, various kinds of improvements considering safety, workability, etc. may be applied to the needle of the present invention. For example, a safety cover is preferably set to lock the needle by the cover so that the needle is not touched after blood collection. In the case of the blood collecting needle, it is preferable that two needle tubes are connected to each other or the needle base is designed to be transparent or translucent so that puncturing into vein is found, that is, so-called flash-back can be visually checked. Furthermore, in order to enhance the workability at the blood collection time, a holder-attached blood collecting needle, a holder-attached winged needle and a holder-attached syringe needle may be designed by integrating a holder of a blood collecting tube with the needle.

(Manufacturing of Needle)

The needle of the present invention is not limited to a special one, and it may be manufactured according to a normal method in this field.

<Vacuum Blood Collecting Tube>

The needle of the present invention may constitute a blood collecting kit together with a vacuum blood collecting tube. Any vacuum blood collecting tube placed on the market may be used as the vacuum blood collecting tube used when blood collection is carried out by the needle of the present invention.

Anticoagulant, antiglycolysis agent, etc. are normally encapsulated in the vacuum blood collecting tubes in advance, and they are selectively used in accordance with the components to be analyzed. Any material which has been hitherto used may be used as blood serum or blood plasma separating medium. For example, synthetic resin having fluidity at ordinary temperature (for example, oligomer of dicyclopentadiene) or the like is added with addition agent such as thixotropy providing agent (for example, condensate of sorbitol and aromatic aldehyde, block copolymer of polyoxyethylene and polyoxylpropylene or the like), gravity adjusting agent (for example, silica), and viscosity adjusting agent (for example, phthalate ester) or the like, and kneaded to achieve thixotropic gel.

As blood anticoagulant, for example, ethylenediaminetetraacetic acid sodium dipotassium salt, ethylenediaminetetraacetic acid sodium tripotassium salt, ethylenediaminetetraacetic acid disodium salt, heparin sodium, heparin lithium, sodium fluoride and citric acid are exemplified.

Any material which has been hitherto used as blood coagulation accelerant may be used as the blood coagulation accelerant, and for example, fine powder of silica may be used.

These chemicals may be inserted in any style such as a powder style, a sheet style, a tablet style or the like. Furthermore, when it is inserted to a flow-in portion 2, it is preferable that they adhere to the inner wall surface of the tube so as to be easily blended with chemicals.

With respect to the amount of blood to be collected by the blood collecting tube, vacuum blood collecting tubes which are different in capacity are selectively used in accordance with an analysis purpose, however, blood collecting tubes of 2mL, 5mL and 10mL in collection amount are generally used. The time required to collect blood is dependent on the condition of an examinee (generally, patient) and the level of skill of an examiner (generally, doctor, nurse, clinical laboratory technologist), and it is normal that the blood collection is completed in several seconds to several tens seconds.

The pressure-reducing degree of the vacuum blood collecting tube on the market ranges from -200mmHg to -650mmHg, and any vacuum blood collecting tube on the market may be used in the blood collecting kit of the present invention. In consideration of the blood collecting speed, etc. , the vacuum blood collecting tube preferably has a pressure-reducing degree of -400mmHg or more, more preferably has a pressure-reducing degree of -450mmHg or more, and most preferably has a pressure-reducing degree of -500mmHg or more. The pressure-reducing degree described here is defined under the condition that the ambient temperature is equal to OmmHg and the vacuum is equal to -760mmHg.

In the present invention, when a blood collecting tube having a small capacity which is suitable for small amount of blood collection is used as a vacuum blood collection tube other than the vacuum collecting tubes on the market, only the amount of blood which is required for test can be collected, and the labor imposed on the examinee can be reduced. The capacity ranged preferably from 0. 3mL to 7mL, more preferably from 0.4mL to 5mL, and most preferably from 0.6mL to 3mL.

<Test Chip>

The needle of the present invention may constitute a test kit together with the test chip. As compared with the blood test using an automatic analysis apparatus which has been hitherto carried out in a medical institution, a test institution or the like, the test based on the test chip can test a larger amount of items with a smaller amount of blood. Assuming that the time required for blood collection is fixed, the inner diameter of the needle of the present invention can be reduced and thus the outer diameter can be reduced as the amount of blood required for the test is reduced. Accordingly, the needle of the present invention is preferably used together with the test chip which can perform tests with a small amount of blood.

The test chip is a compact chip type device in which in order to measure various kinds of components contained in blood, an analyte such as blood or the like is made to flow through a fluid path having a minute cross section by using a capillary phenomenon or electrophoretic migration to be reacted with reagent, and then the respective components in the blood are separated and subjected to transmission spectroscopic analysis, or subjected to light emission reaction with reagent to conduct spectroscopic analysis on emitting light.

<Collected Blood Amount>

Furthermore, according to the present invention, when the blood collection is carried out by using the needle and the vacuum blood collecting tube of the present invention, the passing speed of all the blood inside the needle is preferably equal to 0.01mL per second or more.

Recently, the blood amount required for the test is smaller, and the minimum amount required to test several items is equal to 100μL. Accordingly, if the minimum amount required for the blood test, 100μL can be secured, the needle is expected to be effective to rare blood test. In this invention, a needle which can collect blood of 10μL per second is desired as a needle which can collect the required blood amount of 100μL per 10 seconds.

<Other Embodiments>

The needle of the present invention may be used in other cases than the case where the needle is punctured into the vein of a human being to achieve blood. Particularly, the needle is effectively used to collect blood from a small animal having narrow blood vessels, and it is also usable in a case where blood is collected from an animal such as a dog, a cat or the like in an animal hospital. Furthermore, the needle may be usable to collect blood from small animals such as rats, mousse, rabbits, etc. in the physical and chemical experiments such as chemistry, biochemistry, biology, etc.

Furthermore, in the blood collection targeting human beings, the needle can be used not only for blood collection which are normally carried out in hospitals, health examination, complete physical examination in which blood collection is normally carried out, but also for blood collection for monitoring health in drugstores, health care shops, etc. As compared with the blood collecting needle for normal blood collection, the syringe needle and the winged needle, the pain can be more greatly reduced, and thus the needle of the present invention is effective to applications such as health check to healthy persons, particularly persons who are anxious about sick although they are apparently healthy, etc. As measurements targeting healthy persons are considered not only general biochemical items/blood count, but also the measurement of fluidity of blood (bicker degree), the measurement of stress marker, etc. The fluidity of blood (bicker degree) can be measured by MC-FAN (produced by MC Research Institute) or the like. The needle of the present invention provides little pain, and thus it is effective to the measurement of a material such as stress marker or the like which may be affected by a stimulus at the blood collecting time.

[Embodiments]

The present invention will be described hereunder by using embodiments, however, the present invention is not limited to the embodiments.

(Manufacturing of Blood Collecting Needle)

A hollow stainless tube placed on the market was cold-rolled, and the needle points at both the ends were processed to be keen-edged by the three-side cutting so that the first grinding angle ranges from 8 degrees to 10 degrees. The needle was joined to the member of a resin needle base for a blood collecting needle by adhesive agent, and then a rubber cover was fitted to the stopper-body puncturing side. A sterilization treatment was conducted by gamma rays, and blood collecting needles a to c of embodiments 1 to 3 and a blood collecting needle g of a comparative example 2 were prepared. The needles of a to c are general blood collecting needles having the straight structure in which the outer diameter and the inner diameter were substantially equal to each other from the tip to the base. The outer diameter, the inner diameter and the length of these blood collecting needles are shown in Table 1.

(Manufacturing of Syringe Needle)

A hollow stainless tube placed on the market was cold-rolled, and the needle points at both the ends were processed to be keen-edged by the three-side cutting so that the first grinding angle ranges from 8 degrees to 10 degrees. The needle was joined to the member of a resin needle base for a syringe needle by adhesive agent, and then a cap was fitted. A sterilization treatment was conducted by gamma rays, and a syringe needle d of an embodiment 4 was prepared. The needle d is a general syringe needle having a straight structure in which the outer diameter and the inner diameter are substantially equal to each other from the tip to the base. The outer diameter, the inner diameter and the length of the syringe needle are shown in the Table 1.

(Manufacturing of Winged Needle)

A hollow stainless tube placed on the market was cold-rolled, and the needle points at both the ends were processed to be keen-edged by the three-side cutting so that the first grinding angle ranges from 8 degrees to 10 degrees. The needle was joined to the member of a resin needle base for a winged needle jointed to a conduit tube at one side thereof by adhesive agent, and then a cap was fitted. A sterilization treatment was conducted by gamma rays, and a winged needle e of an embodiment 5 was prepared. The needle e is a general winged needle having a straight structure in which the outer diameter and the inner diameter are substantially equal to each other from the tip to the base. The outer diameter, the inner diameter and the length of the syringe needle are shown in the Table 1.

(Blood Collecting Needle on the market)

In the comparative example 1, a blood collecting needle f of 21G on the market which is conformed with ISO962 1991 was prepared. The outer diameter, the inner diameter and the length of the blood collecting needle are shown in the Table 1. The dimensions of the outer diameter and the inner diameter were measured by cutting the needle tube, picking up an image of the section thereof with an optical microscope (50 times in magnification), and measuring the dimensions from the image. These blood collecting needle were subjected to the sterilization treatment using gamma rays and cleaned.

Table 1

| Specification of Respective Needles | | | | | |
|---|---|---|---|---|---|
| BLOOD COLLECTING NEEDLE | TYPE OF NEEDLE | OUTER DIAMETER [mm] | INNER DIAMETER [mm] | LENGTH [mm] HUMAN-BODY PUNCTURING SIDE | STOPPER-BODY PUNCTURING SIDE |
| EMBODIMENT 1 | MANUFACTURED BLOOD COLLECTING NEEDLE a | 0.50 | 0.38 | 25 | 15 |
| EMBODIMENT 2 | MANUFACTURED BLOOD COLLECTING NEEDLE b | 0.50 | 0.30 | 25 | 15 |
| EMBODIMENT 3 | MANUFACTURED BLOOD COLLECTING NEEDLE c | 0.40 | 0.30 | 19 | 15 |
| EMBODIMENT 4 | MANUFACTURED BLOOD COLLECTING NEEDLE d | 0.50 | 0.38 | 19 | - |
| EMBODIMENT 5 | MANUFACTURED BLOOD COLLECTING NEEDLE e | 0.50 | 0.38 | 19 | - |

(continued)

| Specification of Respective Needles | | | | | |
|---|---|---|---|---|---|
| BLOOD COLLECTING NEEDLE | TYPE OF NEEDLE | OUTER DIAMETER | INNER DIAMETER | LENGTH [mm] | |
| | | [mm] | [mm] | HUMAN-BODY PUNCTURING SIDE | STOPPER-BODY PUNCTURING SIDE |
| COMPARATIVE EXAMPLE 1 | BLOOD COLLECTING NEEDLE f (21g) ON THE MARKET | 0.81 | 0.56 | 38 | 15 |
| COMPARATIVE EXAMPLE 2 | MANUFACTURED BLOOD COLLECTING NEEDLE g | 0.31 | 0.13 | 16 | 15 |

(Pain at the puncturing time)

Pain at the puncturing time was estimated by using the blood collecting needle, the syringe needle and the winged needle of the embodiments 1 to 5 and the comparative examples 1 and 2 . The needles were once punctured into cephalic vein near an elbow for each of ten able-bodied men as examinees one by one. The puncturing was carried out in order of f, a, b, c, d, e, g, and the pain was estimated on the basis of the pain of the blood collecting needle f of 21G placed on the market by ten levels, and the level is reduced as the pain is smaller. The average values of scopes of ten persons are shown in Table 2.

(Estimation of Blood Collecting Time)

The vacuum blood collection was conducted on five men and five women by using the needles of the embodiments 1 to 5 and the comparative examples 1 and 2, and the blood collection time was measured. A vacuum blooding tube produced by NIPRO company "neo tube PET vacuum blood collecting tube" using heparin lithium as anticoagulant of 5mL in capacity and 4mL in collected blood amount was used for blood collection. The syringe needle and the winged needle were applied to vacuum blood collection by using a lure adaptor.
The needle was fitted to "one-touch holder N3" produced by NIPRO company, and punctured into the vein of the blood collection targets. The vacuum blood collecting tube was inserted into the stopper-body puncturing needle tube side (lure adaptor side) and the blood collection was started. The time period from the time at which the flow-in of blood was started till the time at which the flow-in of blood was stopped was measure to measure the blood collecting time. The average value of the ten persons were set as the blood collection time. If the collection time is within 20 seconds, it is estimated as A, if it ranges from 20 seconds to 40 seconds, it is estimated as B, if it ranges from 40 seconds to 60 seconds, it is estimated as C, and if it is equal to 60 seconds or more, it is estimated as D. The result is shown in the Table 2. In this case, the blood collection time was based on the blood collection tube having the blood collection amount of 4mL. However, if a blood collecting tube whose blood collection amount is equal to 2mL is used, the blood collecting time can be reduced to about 1/2, and if the blood collection amount is set to 1mL, the blood collecting time can be reduced to 1/4. As described above, in order to shorten the blood collecting time in accordance with the blood collection amount, the method of adjusting the blood collection amount by changing the pressure-reducing degree is not adopted, but the capacity of the tube itself must be reduced.

Table 2

| Estimation Result of Respective Needles | | | |
|---|---|---|---|
| BLOOD COLLECTING NEEDLE | TYPE OF NEEDLE | PAIN | BLOOD COLLECTING TIME |
| EMBODIMENT 1 | MANUFACTURED BLOOD COLLECTING NEEDLE a | 4.5 | B |

(continued)

| Estimation Result of Respective Needles | | | |
|---|---|---|---|
| BLOOD COLLECTING NEEDLE | TYPE OF NEEDLE | PAIN | BLOOD COLLECTING TIME |
| EMBODIMENT 2 | MANUFACTURED BLOOD COLLECTING NEEDLE b | 4.3 | C |
| EMBODIMENT 3 | MANUFACTURED BLOOD COLLECTING NEEDLE c | 4.0 | C |
| EMBODIMENT 4 | MANUFACTURING SYRINGE NEEDLE d | 4.5 | B |
| EMBODIMENT 5 | MANUFACTURED WINGED NEEDLE e | 4.3 | C |
| COMPARATIVE EXAMPLE 1 | BLOOD COLLECTING NEEDLE f (21G) ON THE MARKET | 10 | A |
| COMPARATIVE EXAMPLE 2 | MANUFACTURED BLOOD COLLECTING NEEDLE g | 3.5 | D |

The needles a to e of the present invention in which the outer diameter $D_1$ satisfies $0.35 \leq D_1 < 0.70$ (equation 1) provide less pain as compared with the conventional needle for blood collection, and also could collect blood without any strength problem of the needle. Furthermore, in the case of 4mL blood collection, the blood collection was completed within 40 second. On the other hand, the blood collecting needle d of 0.81mm in outer diameter which has been hitherto used had a large outer diameter, and thus it provided great pain. Furthermore, the blood collecting needle of 0.31 in outer diameter has narrow and thus needed a long blood collecting time.

Furthermore, comparing the blood collecting needles having the same outer diameter according to the embodiments 1 and 2, the blood collecting needle of the embodiment 1 satisfying $0.9 \times D_1 - 0.1 \leq D_2 \leq 0.9 \times D_1 - 0.04$ (equation 2) was shorter in blood collecting time.

According to the present invention, there can be provided a blood collecting needle, a syringe needle and a winged needle that provide less pain as compared with conventional blood collecting needles. Furthermore, the needle having the dimension that satisfies both the blood collecting time and the strength can be efficiently designed.

According to the present invention, there can be provided a blood collecting needle that can reduce pain at the blood collection time as compared with a normal blood collecting needle. Furthermore, there can be provided a method of efficiently designing a blood collecting needle, a syringe needle and a winged needle in which the flow-in speed of blood is high with suppressing pain.

The entire disclosure of each and every foreign patent application from which the benefit of foreign priority has been claimed in the present application is incorporated herein by reference, as if fully set forth.

**Claims**

1.  A blood collecting needle, which comprises:

    a needle tube having a human-body puncturing needle tube (1) and a stopper-body puncturing needle tube (3),

    wherein the needle tube has an outer diameter having substantially the same dimension from a tip (5) of the human-body puncturing needle tube (1) to a tip (6) of the stopper-body puncturing needle tube (3),
    wherein the needle tube has an inner diameter having substantially the same dimension from the tip (5) of the human-body puncturing needle tube (1) to the tip (6) of the stopper-body puncturing needle tube (3), and
    wherein the blood collecting needle satisfies the following equation:

$$0.35 \leq D_1 < 0.70,$$

    wherein $D_1$ represents the outer diameter of the needle tube.

**2.** The blood collecting needle according to claim 1, which satisfies the following equation:

$$0.9 \times D_1 - 0.1 \leq D_2 \leq 0.9 \times D_1 - 0.04,$$

wherein $D_1$ represents the outer diameter of the needle tube; and
$D_2$ represents the inner diameter of the needle tube.

**3.** The blood collecting needle according to claim 1 or claim 2, which further comprises:

   a needle base (2),

wherein the needle base (2) has a mark indicating an orientation of a blade.

**4.** The blood collecting needle according to any one of claims 1-3, which satisfies the following equation:

$$0.9 \times D_1 - 0.09 \leq D_2 \leq 0.9 \times D_1 - 0.05,$$

wherein $D_1$ represents the outer diameter of the needle tube; and
$D_2$ represents the inner diameter of the needle tube.

**5.** A syringe needle, which comprises:

   a needle tube (12) having a tip and a base (11),

wherein the needle tube (12) has an outer diameter having substantially the same dimension from the tip to the base (11) of the needle tube (12),
wherein the needle tube (12) has an inner diameter having substantially the same dimension from the tip to the base (11) of the needle tube (12), and
wherein the syringe needle satisfies the following equations:

$$0.35 \leq D_1 < 0.70,$$

and

$$0.9 \times D_1 - 0.1 \leq D_2 \leq 0.9 \times D_1 - 0.04,$$

wherein $D_1$ represents the outer diameter of the needle tube (12); and
$D_2$ represents the inner diameter of the needle tube (12).

**6.** A winged needle, which comprises:

   a needle tube (21) having a tip and a base (22),

wherein the needle tube (21) has an outer diameter having substantially the same dimension from the tip to the base (22) of the needle tube (21),
wherein the needle tube (21) has an inner diameter having substantially the same dimension from the tip to the base (22) of the needle tube (21), and
wherein the winged needle satisfies the following equation

$$0.35 \leq D_1 < 0.70,$$

and

$$0.9 \times D_1 - 0.1 \leq D_2 \leq 0.9 \times D_1 - 0.04,$$

wherein $D_1$ represents the outer diameter of the needle tube (21); and
$D_2$ represents the inner diameter of the needle tube (21).

7. A test kit, which comprises:

   a blood collecting needle according to any one of claims 1-4; and
   a test chip.

8. A blood collecting kit, which comprises:

   a blood collecting needle according to any one of claims 1-4; and
   a vacuum blood collecting tube.

9. The blood collecting kit according to claim 8,
   wherein the vacuum blood collecting tube has a content amount of 2.5 ml or less.

## FIG. 1

## FIG. 2

EP 1 834 582 A1

*FIG. 3*

# FIG. 4

*FIG. 5A*

*FIG. 5B*

European Patent
Office

**EUROPEAN SEARCH REPORT**

Application Number

EP 07 10 4285

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/183697 A1 (ALEXANDER GARY E [US] ET AL) 5 December 2002 (2002-12-05) * figure 6d * * paragraph [0045] * | 1 | INV. A61B5/15 |
| X | US 5 645 537 A (POWLES TREVOR J [GB] ET AL) 8 July 1997 (1997-07-08) * figures 1,3 * * column 3, line 50 - line 56 * | 5,6 | |
| X | WO 99/13928 A (ONCOTECH INC [US]) 25 March 1999 (1999-03-25) * figure 14 * * page 6, line 6 - line 20 * * page 15, line 23 - line 25 * | 2-4,7-9 | |
| X | ANONYMOUS: "Hypodermic Needle Gauge Chart" INTERNET ARTICLE, [Online] 10 October 2004 (2004-10-10), XP002441143 Retrieved from the Internet: URL:http://www.medtube.com/hypo_chrt.htm> [retrieved on 2007-07-01] * 27 Gauge, XTW * | 6 | TECHNICAL FIELDS SEARCHED (IPC) A61B A61M |

The present search report has been drawn up for all claims

| Place of search | Date of completion of the search | Examiner |
|---|---|---|
| The Hague | 6 July 2007 | Bengtsson, Johan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 834 582 A1

## ANNEX TO THE EUROPEAN SEARCH REPORT
## ON EUROPEAN PATENT APPLICATION NO.

EP 07 10 4285

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2007

| Patent document cited in search report | | Publication date | Patent family member(s) | | Publication date |
|---|---|---|---|---|---|
| US 2002183697 | A1 | 05-12-2002 | NONE | | |
| US 5645537 | A | 08-07-1997 | US | 5330443 A | 19-07-1994 |
| WO 9913928 | A | 25-03-1999 | AU | 9568398 A | 05-04-1999 |
| | | | CA | 2303787 A1 | 25-03-1999 |
| | | | EP | 1035881 A1 | 20-09-2000 |
| | | | JP | 2001516622 T | 02-10-2001 |

EPO FORM P0459

For more details about this annex : see Official Journal of the European Patent Office, No. 12/82

20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004041391 A
- JP 2065870 A
- JP 2001527435 T